# EUROPEAN PATENT SPECIFICATION

(11) **EP 3 130 369 B1**
(45) Date of publication and mention of the grant of the patent: **09.10.2019**
(21) Application number: 15777494.4
(22) Date of filing: 25.03.2015
(51) Int. Cl.: A61M 5/315, H04M 1/00, H04M 11/00, G16H 10/60, A61M 5/142

(54) **PHARMACEUTICAL INJECTION DEVICE, AND MEDICAL SUPPORT SYSTEM AND MEDICAL SUPPORT METHOD EMPLOYING SAME**
PHARMAZEUTISCHES INJEKTIONSGERÄT, UND MEDIZINISCHES UNTERSTÜTZUNGSSYSTEM UND MEDIZINISCHE UNTERSTÜTZUNGSMETHODE, WELCHE SELBIGES BENUTZEN
DISPOSITIF PHARMACEUTIQUE D'INJECTION, ET SYSTÈME MÉDICAL DE SUPPORT ET MÉTHODE MÉDICALE DE SUPPORT L'UTILISANT

(30) Priority: 08.04.2014 JP 2014079555
(43) Date of publication of application: 15.02.2017
(73) Proprietor: PHC Holdings Corporation, Tokyo 105-8433 (JP)
(72) Inventor: IWASAKI, Masahiro, Toon-shi, Ehime 791-0395 (JP)
(74) Representative: Eisenführ Speiser
(86) International application number: PCT/JP2015/059158
(87) International publication number: WO 2015/156134

(56) References cited:
- WO-A1-2005/041432
- WO-A1-2010/075157
- JP-A- 2004 344 649
- JP-A- 2010 505 593
- JP-A- 2013 537 844
- US-A1- 2006 036 134
- US-A1- 2008 086 086
- US-A1- 2011 124 996
- US-B2- 7 231 263

## Description

### TECHNICAL FIELD

The present invention relates to a pharmaceutical injection device that injects growth hormone or another such pharmaceutical, for example, and to a medical support system in which this device is used.

### BACKGROUND ART

For example, a pharmaceutical injection device that injected growth hormone or the like had a configuration comprising a main body case, a piston that pushed the pharmaceutical contained in this main body case out through an injection needle and into the patient's body, a drive motor that drives the piston, a first controller connected to this drive motor, and a memory connected to this first controller.

Since data related to the pharmaceutical injection amount set by a physician, for example, is stored in the memory of the pharmaceutical injection device, when the patient actuates the drive motor, the piston is driven on the basis of this data and the set pharmaceutical is injected into the patient's body.

Since the data related to the pharmaceutical injection amount is set by a physician or the like, the patient must bring the pharmaceutical injection device to the physician's office when receiving treatment, but patients often forget to bring the pharmaceutical injection device, in which case the data cannot be changed until the patient sees the physician the next time.

For example, a patient who injects growth hormone will normally go the doctor only once every few months, and this is undesirable because the above-mentioned data change will not be done until several months later.

In view of this, it has been proposed that this data change be executed via a mobile communication device.

Specifically, the configuration is such that when change data is transmitted wirelessly from the doctor's medical equipment input apparatus to the patient's mobile communication device, this mobile communication device can immediately transmit data for changing the pharmaceutical injection amount to the pharmaceutical injection device (as conventional art related to this, see, for example, Patent Literature 1 below).

US 2008/0086086 discloses a system for infusing liquid to a body includes an infusion device, a network interface with a cell phone and wireless link and a network server system capable of communication with the infusion device through the network interface. The server system has access to a file of information specific to the controller for the infusion device. The infusion device includes a source of infusion fluid, a delivery port, a pump between the source of infusion fluid and the delivery port and a controller capable of programmable pump rate and sequence.

US 7,231,263 B2 discloses a method for controlling an insulin pump through the Internet. The method comprises the steps of ascertaining whether a logged-in person is a physician; determining the logged-in person as a nurse when the logged-in person is not a physician, receiving a patient's blood sugar level data and generating a command to change an insulin injection amount; checking whether the logged-in person is an attending physician when the logged-in person is a physician, and changing the logged-in person to an attending physician when the logged-in person is not an attending physician; and driving a corresponding insulin pump having the patient's ID by transmitting through the Internet and Bluetooth modules a new prescription made in consideration of a current blood sugar level measurement and insulin injection amount, when the logged-in person is an attending physician or when the logged-in person is changed to an attending physician.

WO 2005/041432 A1 discloses a method for controlling an insulin pump through the Internet. The method comprises the steps of ascertaining whether a logged-in person is a physician; determining the logged-in person as a nurse when the logged-in person is not a physician, receiving a patient's blood sugar level data and generating a command to change an insulin injection amount; checking whether the logged-in person is an attending physician when the logged-in person is a physician, and changing the logged-in person to an attending physician when the logged-in person is not an attending physician; and driving a corresponding insulin pump having the patient's ID by transmitting through the Internet and Bluetooth modules a new prescription made in consideration of a current blood sugar level measurement and insulin injection amount, when the logged-in person is an attending physician or when the logged-in person is changed to an attending physician.

WO 2010/075157 A1 discloses an infusion system that includes a controller device (610) and a communication system to provide for two-way communication between the controller device and an infusion device that controls delivery of fluids to a user's body. Either the controller device or the infusion device may be integrated with a characteristic determining device in a single housing. The housing, in turn, may include a test-strip receptacle (G20) and an illuminator (671) disposed so as to illuminate an area covering the receptacle and a test-strip inserted therein. The illuminator may be configured to be activated automatically when a test strip is inserted into the receptacle, selectively by the user via a button, key, or similar mechanism, and/or when the ambient light level, measured, e.g., with a light sensor, falls below a predetermined intensity. The illumina tor may be a LED emitting white light, and may provide illumination at various levels of intensity.

US 2011/0124996 A1 Diabetes health management systems for use in portable devices having and methods thereof having a user interface, a processor, a memory, and a communication circuit are disclosed. In one embodiment, a diabetes health management system has program code further including a communications module, a data module, a therapy module, and an analysis module. The communications module wirelessly couples the portable device to a plurality of user devices. The data module receives and stores into the memory blood glucose measurement values, insulin dosage data, and health data entries. The therapy module determines a therapy advice message based at least in part on the received blood glucose measurement values, the received insulin dosage data, and displays the therapy
advice message on the user interface. The analysis module displays on the user interface a graphical representation of selected blood glucose measurement values, selected insulin dosage data, selected health data entries, or combinations thereof.

### CITATION LIST

### PATENT LITERATURE

Patent Literature 1: Japanese Laid-Open Patent Application 2004-344649

### SUMMARY

### TECHNICAL PROBLEM

The above-mentioned conventional art involves measuring a blood glucose level and then changing the insulin injection amount in real time on the basis of this blood glucose level. To this end, the mobile communication device and the pharmaceutical injection device must be kept in a communicable state at all times, and as a result, the power consumption expense of the pharmaceutical injection device rises. Accordingly, a power supply with a large capacity must be used with the pharmaceutical injection device. As a result, the pharmaceutical injection device ends up being larger, which makes it less convenient to use. It is therefore an object of the present invention to make such a device easier to use.

### SOLUTION TO PROBLEM

To achieve this object, the present invention is configured to comprise a main body case, a piston that pushes a pharmaceutical contained in the main body case out through an injection needle and into the patient's body, a drive motor that drives the piston, a first controller that is connected to the drive motor, a memory and a first near field communication component that are connected to the first controller, and a battery that supplies power to the drive motor, the first controller, the memory, and the first near field communication component, wherein the first controller receives change data pertaining to the amount of drive by the drive motor from a mobile communication device through the first near field communication component, records the change data in the memory, and sends the execution of change data, as the change data reception status, back to the mobile communication device through the first near field communication component, thus achieving the stated object. Specifically, with the present invention the configuration is such that change data for the drive amount by the drive motor is received from a mobile communication device via a near field communication component, this change data is recorded in a memory, and the information indicating the storage of the change data is sent as the change data reception status back to the mobile communication device via the near field communication component, the information is transmitted from the mobile communication device to the medical equipment input apparatus with the second communication component and the first communication component, so there is no need for connecting to the mobile communication device in real time.

Therefore, the pharmaceutical injection device consumes less power, so there is no need for a large-capacity battery to be used with the pharmaceutical injection device, and as a result the pharmaceutical injection device can be more compact, which makes it easier to use.

### ADVANTAGEOUS EFFECTS

The present invention provides a pharmaceutical injection device that is compact and easy to use.

### BRIEF DESCRIPTION OF DRAWINGS

FIG. 1 shows a pharmaceutical injection device pertaining to an embodiment of the present invention, and a medical support system in which this device is used;
FIG. 2 is an oblique view of the pharmaceutical injection device in FIG. 1;
FIG. 3 is an oblique view of the pharmaceutical injection device in FIG. 1;
FIG. 4 is a cross section of the pharmaceutical injection device in FIG. 2;
FIG. 5 is a control block diagram of the pharmaceutical injection device in FIG. 2;
FIG. 6 is a control block diagram of the mobile communication device 2 in FIG. 1;
FIG. 7 is a control block diagram of the personal computer in FIG. 1;
FIG. 8 shows the display on the personal computer in FIG. 1;
FIG. 9 shows the display on the personal computer in FIG. 1;
FIG. 10 is an operational flowchart of the mobile communication device 2 in FIG. 1;
FIG. 11 shows the display component of the mobile communication device 2 in FIG. 1;
FIG. 12 shows the display component of the mobile communication device 2 in FIG. 1;
FIG. 13 shows the display component of the mobile communication device 2 in FIG. 1;
FIG. 14 shows the display component of the mobile communication device 2 in FIG. 1;
FIG. 15 is an operational flowchart of the mobile communication device 2 and pharmaceutical injection device 1 in FIG. 1;
FIG. 16 shows the display component of the personal computer in FIG. 1; and
FIG. 17 shows the display component of the personal computer in FIG. 1.

### DESCRIPTION OF EMBODIMENTS

Embodiments of the present invention will now be described in detail through reference to the drawings.

### Embodiment 1

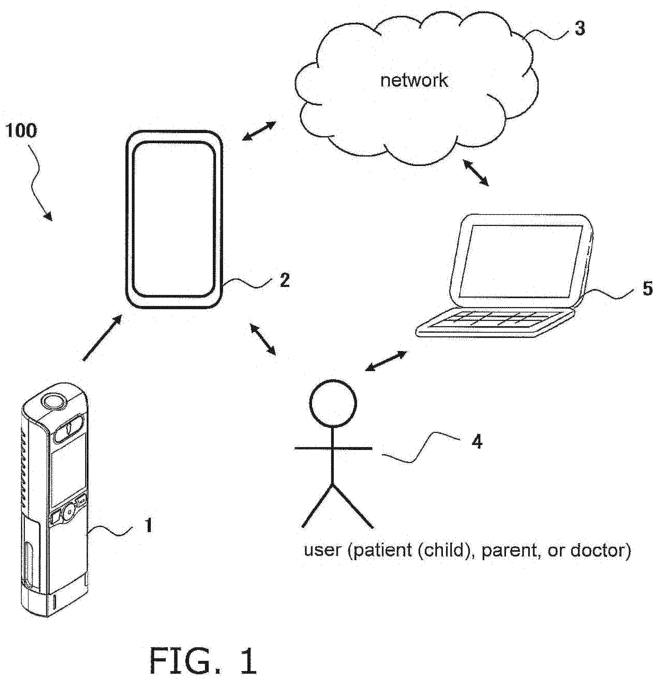

FIG. 1 shows the medical support system in an embodiment of the present invention. The medical support system 100 in this embodiment comprises a pharmaceutical injection device 1 (an example of medical equipment used by a patient 4), a mobile communication device 2 that acquires usage information about this pharmaceutical injection device 1, and a personal computer 5 (an example of a medical equipment input apparatus) to which the usage information about the pharmaceutical injection device 1 is transmitted from the mobile communication device 2 via a network 3.

### Pharmaceutical Injection Device

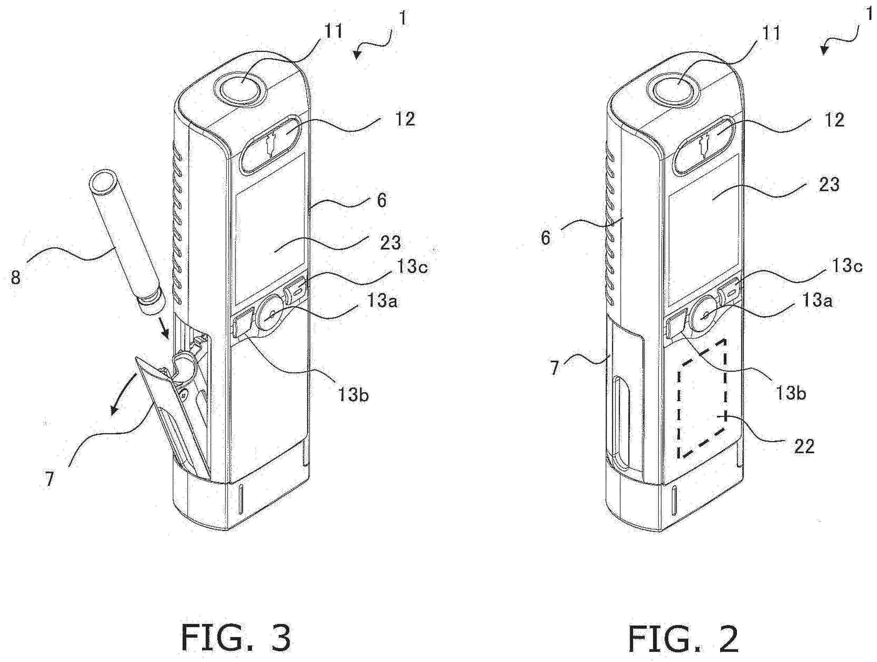

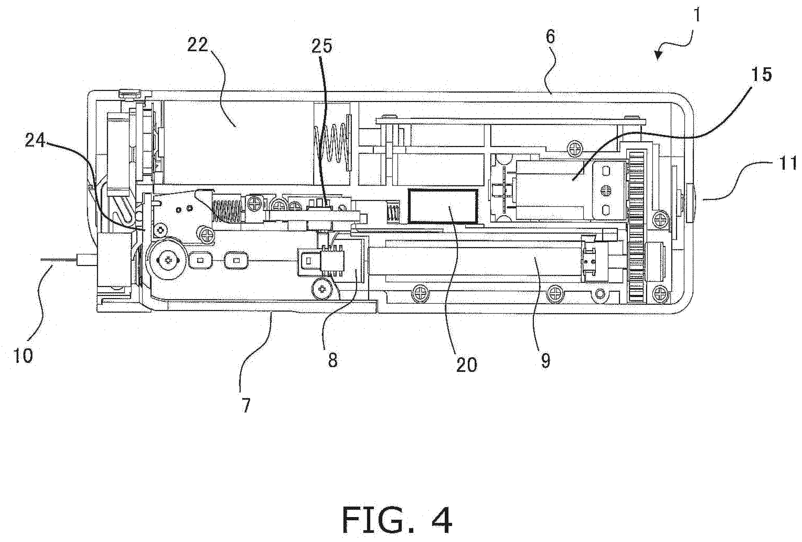

As shown in FIGS. 2 and 3, the pharmaceutical injection device 1 is designed so that the state in FIG. 4 results when a cover 7 of a main body case 6 is opened up, a pharmaceutical syringe 8 is mounted, and an injection needle 10 is mounted to the distal end of this pharmaceutical syringe 8.

That is, in the state of the pharmaceutical injection device 1 shown in FIG. 4 (in which the pharmaceutical syringe 8 is mounted and the cover 7 is closed), the pharmaceutical inside the pharmaceutical syringe 8 can be injected from the injection needle 10 at the distal end into the patient's body by pushing a piston 9 into the pharmaceutical syringe 8.

In this embodiment, the pharmaceutical contained in the pharmaceutical syringe 8 is, for example, a pharmaceutical such as a growth hormone that is injected by the patient 4 (FIG. 1), who suffers from growth hormone deficiency dwarfism, into his own body.

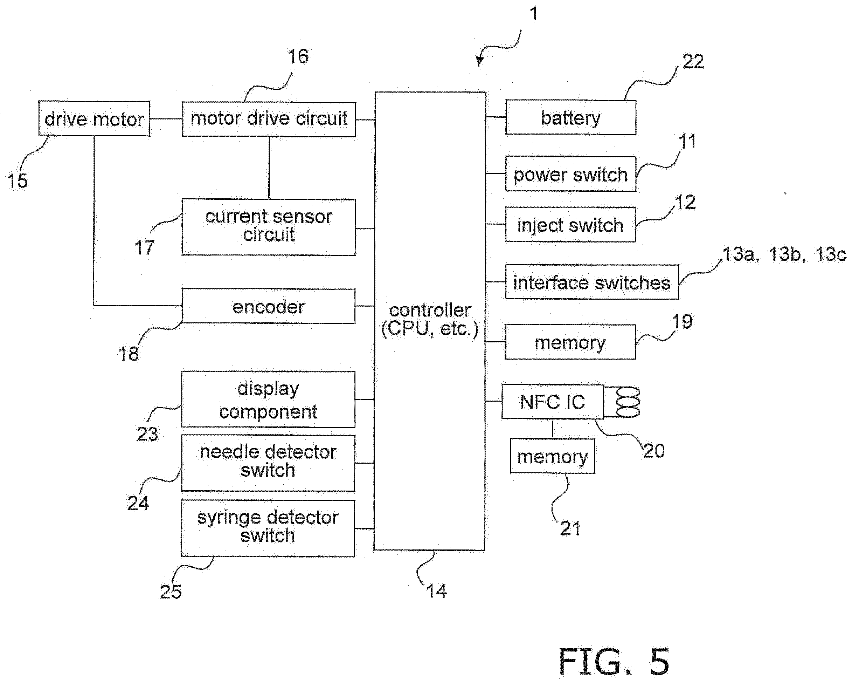

In order to perform this operation of injecting a growth hormone, this pharmaceutical injection device 1 comprises a power switch 11, an inject switch 12, an OK button 13a, a down button 13b, and an up button 13c. As shown in FIG. 5, these are configured to be connected to a controller 14.

A drive motor 15 for driving the piston 9 is also connected to this controller 14 via a motor drive circuit 16.

A current sensing circuit 17 is connected to the motor drive circuit 16, and this current sensing circuit 17 is also connected to the controller 14.

This current sensing circuit 17 detects a major change in the drive current of the motor drive circuit 16 supplying drive current to the drive motor 15, when a large load is applied to the drive motor 15 for one reason or another, or when the drive motor 15 malfunctions, etc. When the current sensing circuit 17 detects an abnormal change in current, the pharmaceutical injection device 1 is configured to notify the controller 14 of this abnormality so that appropriate action can be taken.

More specifically, when an over-current or other such abnormal current change is detected by the current sensing circuit 17, this signal is sent to the controller 14. Upon inputting this, the controller 14 issues a command such as an emergency stop to the motor drive circuit 16, and having receiving this emergency stop command, the motor drive circuit 16 subjects the driver motor 15 to an emergency stop.

Also, an encoder 18 is connected to the drive motor 15, and this encoder 18 is also connected to the controller 14.

That is, the operation of the piston 9 is executed by the drive motor 15 while information about the position of the piston 9 is checked by the encoder 18.

A memory 19 that holds operating programs is also connected to the controller 14.

Furthermore, a communication component 20 that performs near field communication (the "NFC IC" in the example in FIG. 5) is connected to this controller 14, and a memory 21 that holds usage information about the pharmaceutical injection device 1 is connected to the communication component 20. 22 in FIG. 5 is a battery used to execute the operation of the various components shown in FIG. 5.

23 is a display component that displays the input method, etc. 24 is a needle detector switch that detects the mounting of the injection needle 10. 25 is a syringe detector switch that detects the mounting of the pharmaceutical syringe 8.

The display component 23, the needle detector switch 24, and the syringe detector switch 25 are each connected to the controller 14.

### Mobile Communication Device

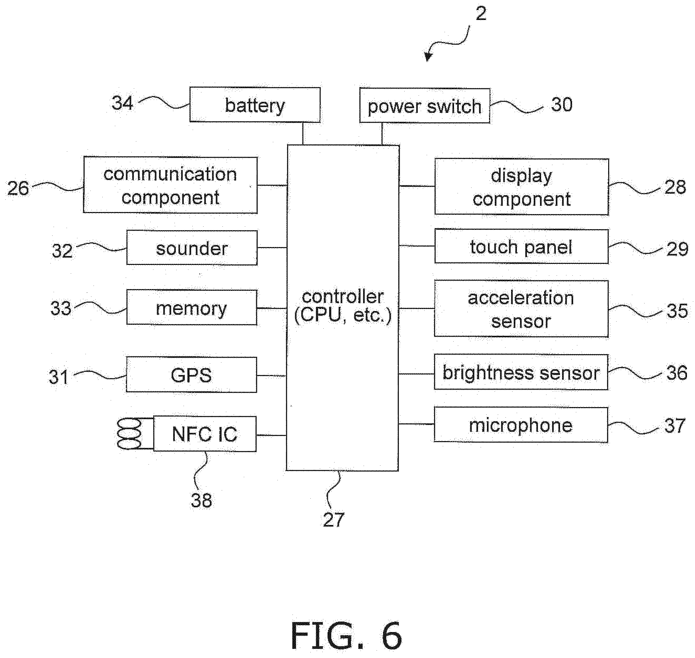

As shown in FIG. 6, the mobile communication device 2 shown in FIG. 1 has a communication component 26 for communicating with a personal computer server (not shown) or another network or for making telephone calls. This communication component 26 is connected to a controller 27.

As is well known, a mobile communication device 2 such as this comprises a display component 28, a touch panel 29 that serves as the interface and is displayed in the display component 28, a power switch 30, a position information sensor 31 (GPS in the example in FIG. 6), a sounder 32 that gives audible output (such as voice output), a memory 33 that holds operating programs for executing the operation of the various components, setting data, usage information for the pharmaceutical injection device 1, and so forth, and a battery 34 for operating the various components.

The display component 28, the touch panel 29, the power switch 30, the position information sensor 31 (GPS in the example in FIG. 6), the sounder 32, and the memory 33 are connected to the controller 27.

Also, an acceleration sensor 35, a brightness sensor 36, a microphone 37, and a communication component 38 that performs near field communication (an NFC IC in the example in FIG. 6) are each connected the controller 27.

The characteristic features of this embodiment, as shown in FIG. 6, is that the communication component 38, which is used for performing near field communication with the communication component 20 of the pharmaceutical injection device 1, is connected to the controller 27 of the mobile communication device 2.

That is, usage information about the pharmaceutical injection device 1 is transferred to the mobile communication device 2 by near field communication (executed by close proximity) between the communication component 20 and the communication component 38 of the mobile communication device 2.

### Personal Computer

The personal computer 5 shown in FIG. 1 will now be described. This personal computer 5 is owned by a doctor.

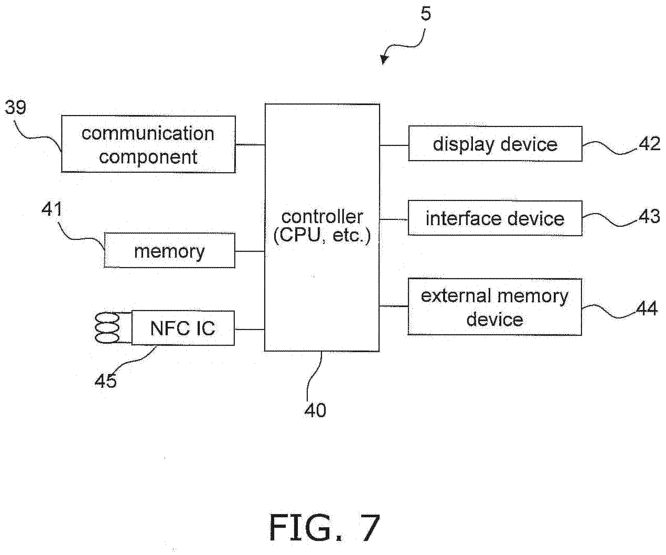

As shown in FIG. 7, the personal computer 5 has a communication component 39 that communicates with the communication component 26 of the mobile communication device 2 via the network 3, etc., a controller 40 that is connected to this communication component 39, and a memory 41 that is connected to this controller 40.

Also, a display device 42, an interface device 43, and an external memory device 44 are connected to the controller 40.

Furthermore, this controller 40 is connected to a communication component 45 (an NFC IC in the example in FIG. 7) for performing near field communication (executed by close proximity) with the communication component 38 of the mobile communication device 2 or near field communication with the communication component 20 of the pharmaceutical injection device 1.

### Medical Support Method

With the above configuration, the drive amount of the drive motor 15 (see FIGS. 4 and 5) of the pharmaceutical injection device 1 is held in the memory 19 (see FIG. 5). This drive amount corresponds to the amount of pharmaceutical to be injected into the patient. That is, the pharmaceutical injection amount for the patient 4 is held in the memory 19, and this injection amount is data set by a doctor at a hospital and stored in the memory 19. The patient 4 injects the pharmaceutical each time by using the pharmaceutical injection device 1 on the basis of the injection amount data set by the hospital doctor and stored in the memory.

Let us say that the patient 4 goes to the doctor once every three months, and after being examined by the doctor, his data is updated by the doctor and stored in the memory 19 according to his physical condition at the time of that visit. Therefore, the patient 4 has to bring the pharmaceutical injection device 1 to the doctor's office for each appointment. However, the patient may sometimes forget to bring the pharmaceutical injection device 1.

In this embodiment, this update data (the newly set pharmaceutical injection amount) is stored by the doctor in the memory 33 (see FIG. 6) of the mobile communication device 2 carried by the patient 4, so the patient 4 can transfer this update data to the pharmaceutical injection device 1 at home, where it can be used as the pharmaceutical injection amount from that point on.

The following description, with reference to the drawings, will be of a method for setting and updating a new pharmaceutical injection amount by a doctor, a method for setting and updating update data for the injection amount in the pharmaceutical injection device 1 by the patient 4 at home after it has been set and updated by the doctor, and so forth.

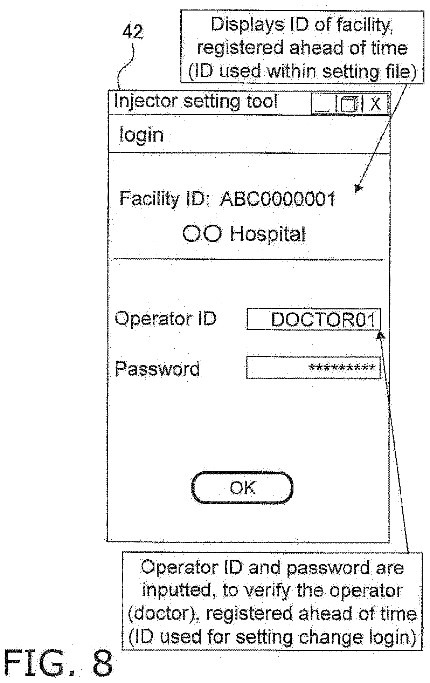

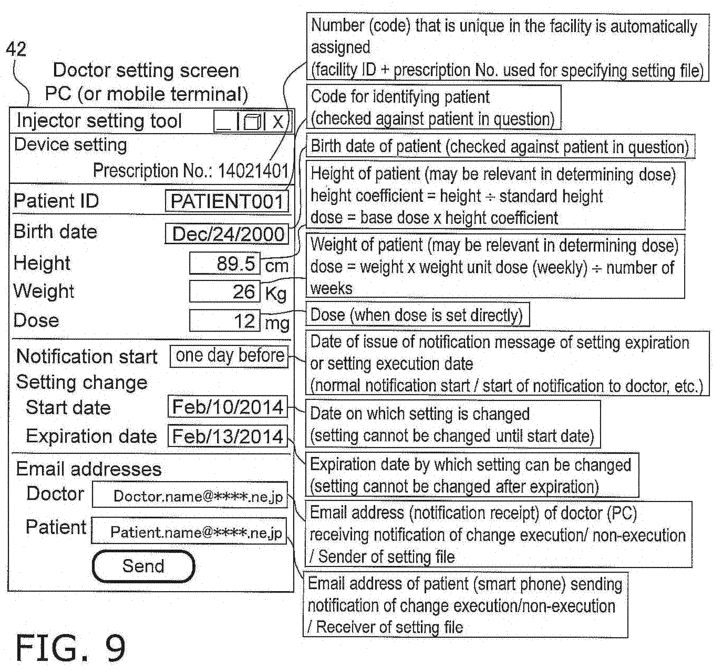

FIGS. 8 and 9 show a state in which the personal computer 5 is used by a doctor at a hospital to update the pharmaceutical injection amount for a patient 4.

First, in FIG. 8, the operator's (in this case, the doctor's) ID and password are inputted using with the display device 42 of the personal computer 5 (see FIG. 7). If the operator ID and password are correct, next, an injector setting tool screen (an equipment setting screen used by the doctor) is displayed on the screen of the display device 42 as shown in FIG. 9.

Patient data is updated on this equipment setting screen by the doctor. That is, as shown in FIG. 9, since the height and weight of a patient 4 vary at the time of examination, the correct pharmaceutical injection amount is changed to match the condition of that patient 4.

There is usually one pharmaceutical injection device 1 for each patient, with that device being used only for that patient.

Next, what is displayed on the injector setting tool screen (an equipment setting screen used by the doctor) of the display device 42 in FIG. 9 will be briefly described.

The injector setting tool screen displays, starting from the top, the "prescription management-use prescription number," "patient ID" (a unique code for identifying the patient 4), "birth date" (the birth date of the patient 4), "height" (data about the height of the patient 4), "weight" (weight data for the patient 4), "dosage" (this indicates the pharmaceutical injection amount), "start of notification" (this indicates the date on which a notification message is given for the setting expiration or the setting implementation date; shown as "one day before" in FIG. 9), "setting change/start date" (this indicates the date when the setting is changed, and no change can be made until this start date), "setting change/expiration date" (this indicates the expiration date by which the setting can be changed, and the setting cannot be changed after this date), "email address: doctor" (the email address of the doctor, which is used to send injection amount setting data and the like and to receive notifications about whether or not to execute setting and changing the injection amount), "email address: patient" (the email address of the patient, which is used to send notifications about whether or not to set and change the injection amount, and to receive injection amount change data and the like from the doctor), and so forth.

The data setting and change of the pharmaceutical dose (injection amount) may involve direct input and setting when the doctor has determined the condition of the patient, or may be performed on the basis of the age, height, and weight of the patient.

In this example, the date on which the setting change can be started is February 10, 2014, and the expiration date by which this change can be made is February 13, 2014. In addition, the email addresses of the doctor and patient 4 are set.

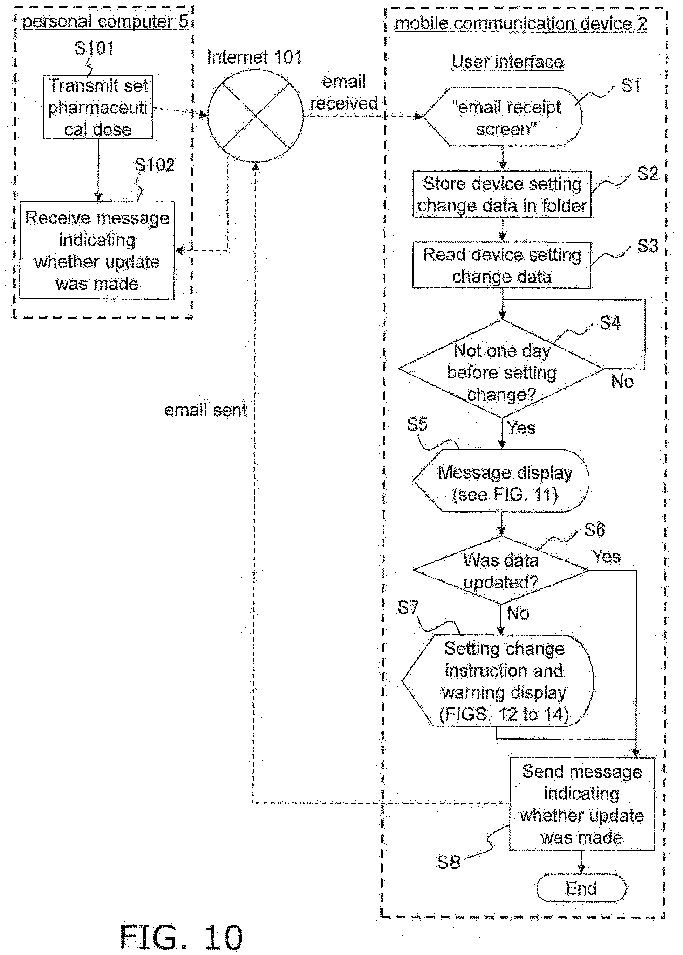

Therefore, the pharmaceutical dose set with the personal computer 5 is sent by email from the personal computer 5 to the mobile communication device 2 of the patient 4 (S101 in FIG. 10). This S101 corresponds to an example of a first transmission step.

Since the operation by which the pharmaceutical dose is sent from the personal computer 5 to the mobile communication device 2 is the same as in the above-mentioned patent literature, it will be omitted here to keep the description from becoming too complicated.

Upon receiving an email, the controller 27 of the mobile communication device 2 causes the display component 28 to display a screen indicating that an email has been received (S1 in FIG. 10).

The controller 27 then puts the equipment setting change data (such as update data about the pharmaceutical injection amount) received in this email in a folder located in the memory 33 of the mobile communication device 2 (S2 in FIG. 10). The controller 27 then reads from this email the changed pharmaceutical dose setting (injection amount), the setting change start date, the setting change expiration date, and so forth (S3 in FIG. 10).

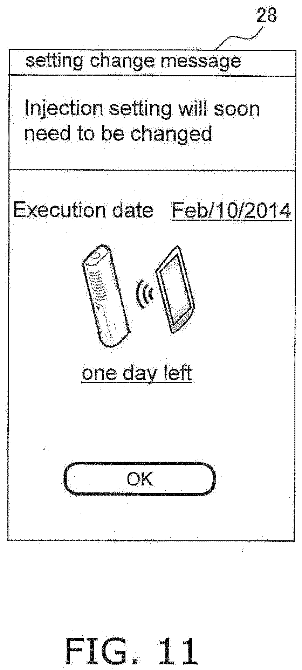

After this, when February 9, 2014 arrives, the controller 27 of the mobile communication device 2 causes the display component 28 to give the display shown in FIG. 11 (S4 in FIG. 10).

That is, as shown in FIG. 9, since the doctor uses the personal computer 5 to set the notification for a setting change to one day before, when February 9 arrives, as shown in FIG. 11, the controller 27 causes the display component 28 to display a message prompting the user to change the setting of the dose (S5 in FIG. 10).

In the example in FIG. 11, the display shows that the date on which the pharmaceutical dose setting is to be changed will be the following day (February 10, 2014).

That is, the patient is notified that starting on February 10, 2014 (the next day), the pharmaceutical dose setting (injection amount) can be changed, and is advised to be ready for this.

Therefore, when February 10, 2014 arrives, the patient 4 brings the mobile communication device 2 into close proximity with the pharmaceutical injection device 1 and executes the above-mentioned change of the pharmaceutical dose. That is, change data about the pharmaceutical injection amount is transferred from the communication component 38 of the mobile communication device 2 to the communication component 20 of the pharmaceutical injection device 1, and this newly changed pharmaceutical injection amount data is stored in the memory 19 (S6 in FIG. 10).

Therefore, from that point on, the pharmaceutical injection device 1 will execute pharmaceutical injection on the basis of the newly changed pharmaceutical dose (injection amount) stored in the memory 19.

Also, the mobile communication device 2 sends the personal computer 5 a notification to the effect that change data for the pharmaceutical injection amount was transferred to the pharmaceutical injection device 1.

Specifically, in this embodiment, change data about a new pharmaceutical injection amount corresponding to the drive amount of the drive motor 15 is received from the mobile communication device 2 via the communication component 20 of the pharmaceutical injection device 1 and the communication component 38 of the mobile communication device 2 (performing near field communication), this change data is recorded in the memory 19 of the pharmaceutical injection device 1, and the result of this change data for the pharmaceutical injection amount being received is sent back to the mobile communication device 2 via the communication components 20 and 38 (performing near field communication). Because of this configuration, there is no need for the pharmaceutical injection device 1 to be connected in real time to the mobile communication device 2.

Therefore, the pharmaceutical injection device 1 consumes less power, so there is no need for a large-capacity battery 22 to be used with the pharmaceutical injection device 1, and as a result the pharmaceutical injection device 1 can be made more compact, which makes it more convenient to use.

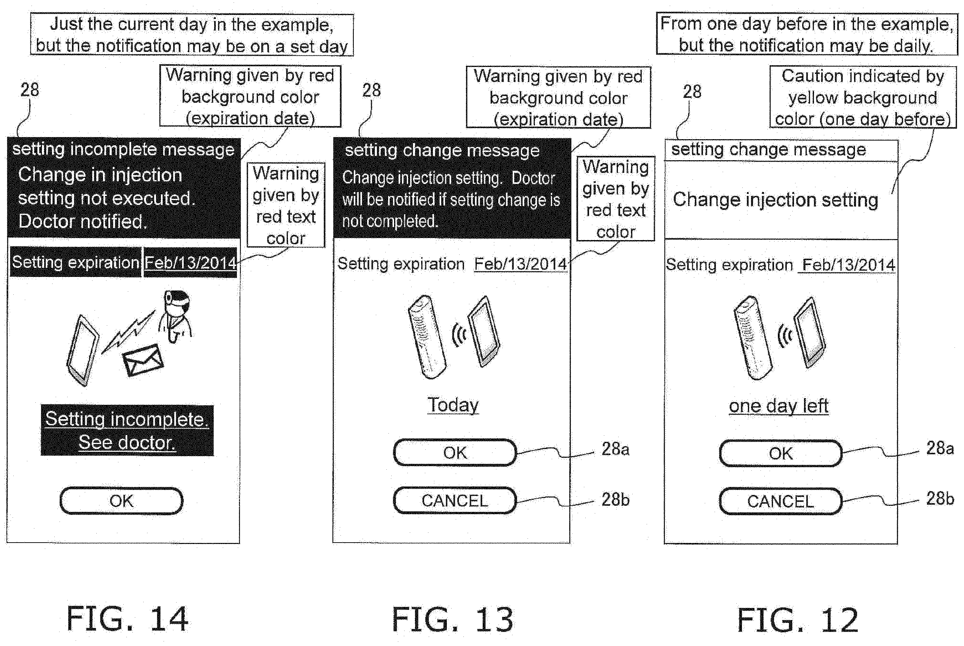

In contrast, if the patient 4 does not send update data for the pharmaceutical injection amount to the pharmaceutical injection device 1 even though the setting change expiration by which data update is to be performed (indicating that the setting change start date for the pharmaceutical dose (the injection amount) has passed) has arrived, the controller 27 of the mobile communication device 2 causes the display component 28 to give the display shown in FIG. 12. As shown in FIG. 12, the display component 28 displays an enter key 28a (also called an OK key) and a cancel key 28b.

That is, in the example in FIG. 9, since the setting start date made by the doctor is February 10, 2014, and the setting change expiration is February 13, 2014, FIG. 12 shows the display state outputted on February 12, 2014. This display includes information indicating that the setting change expires on February 13, and that the setting can be changed for only one more day, and prompts the user to move the mobile communication device 2 and the pharmaceutical injection device 1 close together to change the setting of the pharmaceutical dose. The display shown in FIG. 12 corresponds to an example of a first message.

The controller 27 of the mobile communication device 2 causes the display component 28 to give the display shown in FIG. 13 on the expiration date (the following day) when update data has not been transferred from the mobile communication device 2 to the pharmaceutical injection device 1 despite the fact that the display in FIG. 12 was shown on the display component 28 (S7 in FIG. 10).

That is, in FIG. 13, the patient 4 is advised that the level of urgency is higher than in the state in FIG. 12, so the display color of the message displayed in the upper part of the display device 42 is changed to a color that stands out more than the display in FIG. 12.

More specifically, the background of the setting change message box is changed to a dark color (such as red or navy blue), and the text is changed to a light color (such as white or a pastel color), so as to make it easy to see at a glance that the setting expiration is approaching. That is, a notification is given by visual effect.

In this display it is shown that the setting change expiration is today (February 13), and that the mobile communication device 2 should be moved close to the pharmaceutical injection device 1.

When February 14 arrives, the setting change expiration has already passed, so the controller 27 causes the display component 28 to give the display shown in FIG. 14. The display in FIG. 14 corresponds to an example of a second message.

That is, the display tells the user that the change data for the pharmaceutical injection amount was not updated within the setting change period, and a notification that the update could not be performed to the doctor by e-mail, and advises the user to go to see the doctor.

A message to the effect that the change data was not updated is then sent from the mobile communication device 2 to the personal computer 5.

On the other hand, if the change data has been updated, the fact that the change data was updated is sent from the mobile communication device 2, through the network 3, to the personal computer 5 (S8 in FIG. 10).

The personal computer 5 receives notice of whether or not the change data was updated, over a network by email or the like (S102 in FIG. 10).

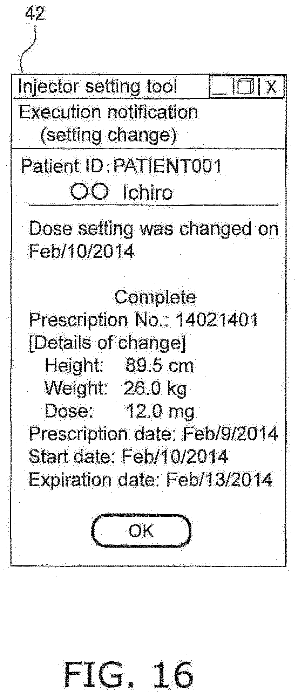

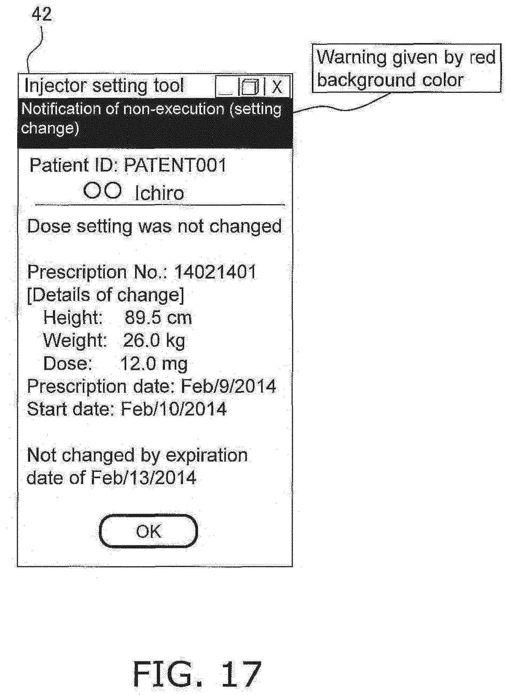

Here, the display content upon receipt by the personal computer 5 is displayed on the display device 42, and an example of this is shown in FIGS. 16 and 17.

Specifically, the display content of the display device 42 shown in FIG. 16 shows the display when change data for the dose has been set in the pharmaceutical injection device 1 used by the patient 4, and displays the patient ID and patient name for the changed dose, the date of the change, patient information, the set dose, and the like, as well as a message to the effect that there has been a change.

The display content of the display device 42 shown in FIG. 17 is displayed when no dose change data was set, and displays the patient ID, patient name, prescription number, information related to change details and the like scheduled to be changed, as well as a message to the effect that there has been a change.

Just as in the case of FIG. 13, the display color of the message display portion is selected to be a color that stands out visually, to tell the patient 4 that action is urgently needed.

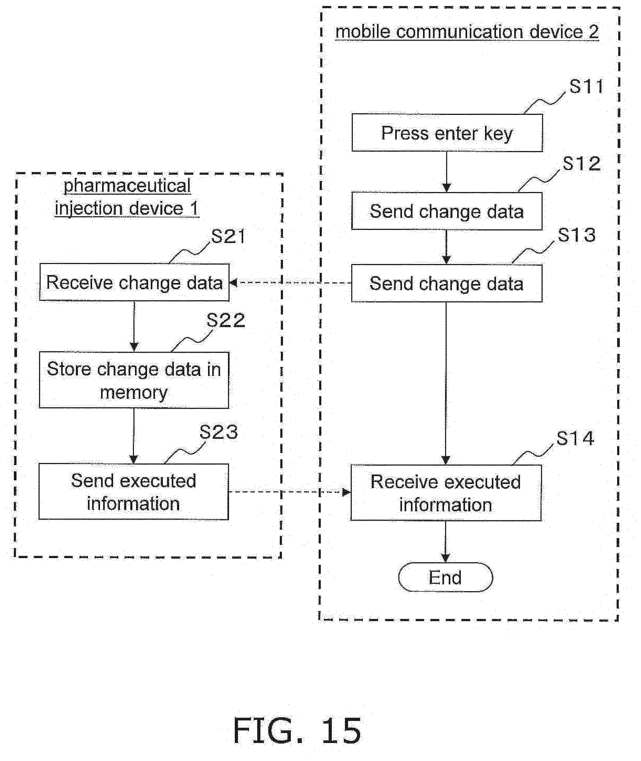

Next, the data update will be described. FIG. 15 is a flowchart of the operation during data update.

To update the data for the pharmaceutical dose, the patient 4 moves the mobile communication device 2 close to the pharmaceutical injection device 1 and hits the enter key 28a (OK key) shown in FIG. 12, whereupon change data about the pharmaceutical dose is sent from the communication component 38 of the mobile communication device 2 to the communication component 20 of the pharmaceutical injection device 1 (S11 in FIG. 15).

S11 in FIG. 15 corresponds to an example of a second transmission step.

When the pharmaceutical injection device 1 receives the change data (S21 in FIG. 15), the change data for the pharmaceutical dose is stored in the memory 19 of the pharmaceutical injection device 1 (S22 in FIG. 15).

S22 in FIG. 15 corresponds to an example of an execution step. Storage in the memory 19 corresponds to an example of the execution of change data.

Next, the pharmaceutical injection device 1 sends information indicating that data was stored in the memory 19 and change data was set back to the mobile communication device 2 (S23 in FIG. 15). S23 in FIG. 15 corresponds to an example of a third transmission step.

Upon receiving the execution information, the mobile communication device 2 determines that the update of data is complete, and as shown in S8 in FIG. 10, information indicating that the update of data is complete is sent over the network 3 to the personal computer 5.

Thus, the mobile communication device 2 can confirm the completion of the update of data in the pharmaceutical injection device 1 by receiving a reply from the pharmaceutical injection device 1, and the doctor can manage the pharmaceutical dose for the patient 4 by sending the confirmation result back to the personal computer 5.

### Main Features

(1) The pharmaceutical injection device 1 in this embodiment comprises the main body case 6, the piston 9, the drive motor 15, the controller 14, the memory 19, the communication component 20, and the battery 22 (see FIGS. 4 and 5).
   The piston 9 pushes the pharmaceutical contained in the main body case 6 out through the injection needle 10 and into the patient's body. The drive motor 15 drives the piston 9. The controller 14 is connected to the drive motor 15.
   The memory 19 is connected to the controller 14. The communication component 20 is connected to the controller 14. The battery 22 supplies power to the drive motor 15, the controller 14, the memory 19, and the communication component 20.
   The controller 14 receives change data for the modification data of the drive motor 15 from the mobile communication device 2 via the communication component 20, records the change data in the memory 19 of the pharmaceutical injection device 1, and sends information about the execution of the change data through the communication component 20 back to the mobile communication device 2, as the reception state of the change data.
   Also, change data can be transmitted by moving the mobile communication device 2 close to the pharmaceutical injection device 1, so the pharmaceutical injection device 1 does not have to be connected in real time to the mobile communication device 2.
   Consequently, the power consumption of the pharmaceutical injection device 1 is far lower than when connected in real time, so there is no need to provide a large-capacity battery to the pharmaceutical injection device 1, and as a result, the pharmaceutical injection device 1 can be made more compact, which improves portability and makes the device more convenient to use.
(2) Also, the medical support method in this embodiment is a medical support method for changing the pharmaceutical injection amount of the pharmaceutical injection device 1, comprising S101 in FIG. 10 (an example of a first transmission step), S13 (an example of a second transmission step), S22 (an example of an execution step), and S23 (an example of a third transmission step) in FIG. 15, and S8 in FIG. 10 (an example of a fourth transmission step).

In S101 in FIG. 10 (an example of a first transmission step), the change data received from the personal computer 5 is transmitted to the mobile communication device 2.

In S13 in FIG. 15 (an example of a second transmission step), change data is transmitted from the mobile communication device 2 to the pharmaceutical injection device 1 by means of near field communication through the communication component 38 of the mobile communication device 2 and the communication component 20 of the pharmaceutical injection device 1.

In S22 in FIG. 15 (an example of an execution step), the setting of change data is executed at the pharmaceutical injection device 1.

In S23 in FIG. 15 (an example of a third transmission step), information about the execution (an example of the execution of change data) of the storage of change data in the memory 19 (of the pharmaceutical injection device 1) by near field communication is transmitted from the pharmaceutical injection device 1 to the mobile communication device 2.

In S8 in FIG. 10 (an example of a fourth transmission step), the execution or non-execution of change data (including whether or not the change data is updated) is transmitted from the mobile communication device 2 to the personal computer 5.

Because change data can be transmitted by moving the mobile communication device 2 close to the pharmaceutical injection device 1, there is no need to connect in real time to the mobile communication device, so the pharmaceutical injection device can be made more compact, which improves its portability and so forth, and makes it more convenient to use.

Also, a health care provider can ascertain whether or not the patient has updated the change data, and can therefore manage the pharmaceutical injection amount.

### Other Embodiments

(A) The above embodiment dealt only with a situation in which the display on the mobile communication device 2, etc., was used to warn or caution the patient 4, but the sounder 32 shown in FIG. 6 can be used to sound an alarm in conjunction with the above-mentioned display, or the timbre can be varied to warn or caution the patient 4 with a hearing effect.
(B) In the above-mentioned embodiment, an example was given of a medical support system that included a pharmaceutical injection device or the like that made use of a growth hormone pharmaceutical, but this is not the only option, and it should go without saying that the present invention can be applied to all cases in which a pharmaceutical is administered at regular intervals, such as for osteoporosis.
(C) Also, in the above description, an example was given of using the pharmaceutical injection device 1, the mobile communication device 2, and the personal computer 5 used by a doctor, but change data can also be sent and received directly between the personal computer 5 and the pharmaceutical injection device 1 through near field communication (NFC). In this case, change data is sent and received by near field communication between the communication component 20 of the pharmaceutical injection device 1 and the communication component 45 of the personal computer 5 (the NFC communication component in the example in FIG. 7).
   In the transmission of change data from the personal computer 5 to the mobile communication device 2, this can be accomplished directly between the communication component 38 of the mobile communication device 2 and the communication component 45 of the personal computer 5 (the NFC communication component in the example in FIG. 7), or the transmission can make use of web content or the like by means of the communication component 26 of the mobile communication device 2 and the communication component 39 of the personal computer 5.
(D) Furthermore, instead of the personal computer 5 used by the doctor, a tablet terminal or a portable communication device can be used to set change data for the pharmaceutical dose.

Here again, the pharmaceutical dose can be easily updated via the communication component 20 of the pharmaceutical injection device 1, and properly injecting the pharmaceutical that needs to be injected regularly, and properly updating the pharmaceutical dose according to that patient's situation contributes to the treatment of the patient, a more compact device with a simple configuration can be obtained, and ease of use by the patient can also be improved.

### INDUSTRIAL APPLICABILITY

With the present invention, the pharmaceutical injection device will consume less power, so there is no need to provide the pharmaceutical injection device with a large-capacity battery, and as a result, the pharmaceutical injection device can be more compact, which makes it more convenient to use.

Therefore, the present invention is expected to find application as a pharmaceutical injection device for injecting growth hormone or other such pharmaceuticals, as well as a medical support system in which this device is used.

### REFERENCE SIGNS LIST

1 pharmaceutical injection device
2 mobile communication device
3 network
4 patient
5 personal computer (an example of a medical equipment input apparatus)
6 main body case
7 cover
8 pharmaceutical syringe
9 piston
10 injection needle
11 power switch
12 inject switch
13a OK button
13b down button
13c up button
14 controller (an example of a first controller)
15 drive motor
16 motor drive circuit
17 current sensing circuit
18 encoder
19 memory
20 communication component (an example of a first near field communication component)
21 memory
22 battery
23 display component
24 needle detector switch
25 syringe detector switch
26 communication component
27 controller (an example of a second controller)
28 display component (an example of a display component)
28a enter key
28b cancel key
29 touch panel
30 power switch
31 position information sensor
32 sounder
33 memory
34 battery
35 acceleration sensor
36 brightness sensor
37 microphone
38 communication component (an example of a second near field communication component)
39 communication component
40 controller
41 memory
42 display device
43 interface device
44 external memory device
45 communication component

## Claims

1. A medical support system, comprising:
a pharmaceutical injection device (1), having a main body case (6); a piston (9) that pushes a pharmaceutical contained in the main body case (6) out through an injection needle (10) and into the patient's body; a drive motor (15) that drives the piston (9); a first controller (14) that is connected to the drive motor (15); a memory (19) that is connected to the first controller (14); a storage recognition component that recognizes that data has been stored in the memory (19); a first near field communication component (20) that is connected to the first controller (14); and a battery (22) that supplies power to the drive motor (15), the first controller (14), the memory (19), and the first near field communication component (20);
a medical equipment input apparatus (5), having a recognition component that recognizes an operator; an input component that inputs change data with respect to a drive amount of the drive motor (15); and a first communication component (39) that sends change data with respect to the drive amount of the drive motor (15) to a mobile communication device (2); and
the mobile communication device (2), having a second near field communication component (38) that performs near field communication with the first near field communication component (20) of the pharmaceutical injection device (1); a second controller (27) that is connected to the second near field communication component (38); a display component (23) that is connected to the second controller (27); and a second communication component (26) that is connected to the second controller (27) and communicates with the medical equipment input apparatus (5),
wherein, with the medical equipment input apparatus (5), the change data can be inputted only by the operator recognized by the recognition component,
the first communication component (39) sends the second communication component (26) change data with respect to the drive amount of the drive motor (15) inputted by the operator,
the first controller (14) of the pharmaceutical injection device (1) receives the change data with respect to the drive amount of the drive motor (15) received by the second communication component (26) of the mobile communication device (2), from the second near field communication component (38) of the mobile communication device (2) via the first near field communication component (20), such that the change data can be transmitted by moving the mobile communication device (2) close to the pharmaceutical injection device (1), so the pharmaceutical injection device (1) does not have to be connected in real time to the mobile communication device (2),
the change data with respect to the drive amount of the drive motor (15) is recorded in the memory (19), this storage in the memory (19) is recognized,
the information indicating the storage of the change data is sent back through the first near field communication component (20) to the mobile communication device (2), the information is transmitted from the mobile communication device (2) to the medical equipment input apparatus (5) with the second communication component (26) and the first communication component (20), and
the second controller (27) sends the medical equipment input apparatus (5) a notification of whether or not to execute near field communication between the first near field communication component (20) of the pharmaceutical injection device (1) and the second near field communication component (38) of the mobile communication device (2).

2. The medical support system according to Claim 1, wherein communication between the first communication component (20) of the medical equipment input apparatus (5) and the second communication component (26) of the mobile communication device (2) is near field communication.

3. The medical support system according to Claim 1, wherein the second controller (27) causes the display component (28) to display a message that reports to the medical equipment input apparatus (5) whether or not to execute near field communication between the first near field communication component (20) of the pharmaceutical injection device (1) and the second near field communication component (38) of the mobile communication device (2).

4. The medical support system according to Claim 1 or 3, wherein the second controller (27) causes the display component (28) to display the date on which the pharmaceutical injection device (1) is scheduled to be changed with the change data for the drive amount of the drive motor (15) received from the medical equipment input apparatus (5).

5. The medical support system according to Claim 4, wherein the second controller (27) causes the display component (28) to display an enter key for executing the update of the change data and the scheduled date of the change.

6. The medical support system according to Claim 5, wherein the second controller (27) causes the display component (28) to display a cancel key for canceling the display of the enter key.

7. The medical support system according to Claim 6, wherein, when near field communication is not executed by the first near field communication component (20) and the second near field communication component (38) on the scheduled date of the change, the second controller (27) causes the display component (28) to display a message notifying the medical equipment input apparatus (5) that near field communication is not executed.

8. A medical support system according to Claim 1,
wherein
the second controller (27) of the mobile communication device (2) sends a notification of whether or not to store the change data, from the mobile communication device (2) to the medical equipment input apparatus (5).

9. A medical support method for changing the pharmaceutical injection amount of a pharmaceutical injection device (1), comprising:
a first transmission step of transmitting change data inputted by an operator recognized at a medical equipment input apparatus (5), to a mobile communication device (2);
a second transmission step of transmitting the change data from the mobile communication device (2) to the pharmaceutical injection device (1) by means of near field communication via a first near field communication component (20) of the pharmaceutical injection device (1) and a second near field communication component (38) of the mobile communication device (1), such that the change data can be transmitted by moving the mobile communication device (2) close to the pharmaceutical injection device (1), so the pharmaceutical injection device (1) does not have to be connected in real time to the mobile communication device (2);
an execution step of the recognizing storage in a memory (19) and setting the change data at the pharmaceutical injection device (1);
a third transmission step of transmitting the medical equipment input apparatus (5) a notification of whether or not to execute near field communication between the first near field communication component (20) of the pharmaceutical injection device (1) and the second near field communication component (38) of the mobile communication device (2);
a forth transmission step of transmitting information indicating that the change data was stored by the near field communication from the pharmaceutical injection device (1) to the mobile communication device (2); and
a fifth transmission step of transmitting storage or non-storage of the change data from the mobile communication device (2) to the medical equipment input apparatus (5).

## Patentansprüche

1. Medizinisches Hilfssystem, umfassend:
eine pharmazeutische Injektionsvorrichtung (1) mit einem Hauptkörpergehäuse (6); einen Kolben (9), der ein in dem Hauptkörpergehäuse (6) enthaltenes Arzneimittel durch eine Injektionsnadel (10) hinaus und in den Körper eines Patienten hinein drückt; einen Antriebsmotor (15), der den Kolben (9) antreibt; eine erste Steuerung (14), die mit dem Antriebsmotor (15) verbunden ist; einen Speicher (19), der mit der ersten Steuerung (14) verbunden ist; eine Speichererkennungskomponente, die erkennt, dass Daten in dem Speicher (19) abgespeichert wurden; eine erste Nahfeldkommunikationskomponente (20), die mit der ersten Steuerung (14) verbunden ist; und eine Batterie (22), die den Antriebsmotor (15), die erste Steuerung (14), den Speicher (19) und die erste Nahfeldkommunikationskomponente (20) mit Strom versorgt;
eine Eingabevorrichtung für medizinische Geräte (5), mit einer Erkennungskomponente, die einen Bediener erkennt; eine Eingabekomponente, die Änderungsdaten in Bezug auf eine Antriebsgröße des Antriebsmotors (15) eingibt; und eine erste Kommunikationskomponente (39), die Änderungsdaten in Bezug auf die Antriebsgröße des Antriebsmotors (15) an eine mobile Kommunikationsvorrichtung (2) sendet; und
die mobile Kommunikationsvorrichtung (2) mit einer zweiten Nahfeldkommunikationskomponente (38), die eine Nahfeldkommunikation mit der ersten Nahfeldkommunikationskomponente (20) der pharmazeutischen Injektionsvorrichtung (1) durchführt; eine zweite Steuerung (27), die mit der zweiten Nahfeldkommunikationskomponente (38) verbunden ist; eine Anzeigekomponente (23), die mit der zweiten Steuerung (27) verbunden ist; und eine zweite Kommunikationskomponente (26), die mit der zweiten Steuerung (27) verbunden ist und mit der Eingabevorrichtung für medizinische Geräte (5) kommuniziert,
wobei mit der Eingabevorrichtung für medizinische Geräte (5) die Änderungsdaten nur von dem von der Erkennungskomponente erkannten Bediener eingegeben werden können,
wobei die erste Kommunikationskomponente (39) der zweiten Kommunikationskomponente (26) vom Bediener eingegebene Änderungsdaten in Bezug auf die Antriebsgröße des Antriebsmotors (15) sendet,
die erste Steuerung (14) der pharmazeutischen Injektionsvorrichtung (1) die Änderungsdaten in Bezug auf die Antriebsgröße des Antriebsmotors (15) empfängt, die von der zweiten Kommunikationskomponente (26) der mobilen Kommunikationsvorrichtung (2) von der zweiten Nahfeldkommunikationskomponente (38) der mobilen Kommunikationsvorrichtung (2) über die erste Nahfeldkommunikationskomponente (20) empfangen wird, so dass die Änderungsdaten durch Bewegen der mobilen Kommunikationsvorrichtung (2) in die Nähe der pharmazeutischen Injektionsvorrichtung (1) übertragen werden können, so dass die pharmazeutische Injektionsvorrichtung (1) nicht in Echtzeit mit der mobilen Kommunikationsvorrichtung (2) verbunden sein muss,
die Änderungsdaten in Bezug auf die Antriebsgröße des Antriebsmotors (15) im Speicher (19) aufgezeichnet werden, wobei dieses Abspeichern im Speicher (19) erkannt wird,
die Informationen, die das Abspeichern der Änderungsdaten anzeigen, über die erste Nahfeldkommunikationskomponente (20) an die mobile Kommunikationsvorrichtung (2) zurückgegeben werden, wobei die Informationen von der mobilen Kommunikationsvorrichtung (2) an die Eingabevorrichtung für medizinische Geräte (5) mit der zweiten Kommunikationskomponente (26) und der ersten Kommunikationskomponente (20) übertragen werden, und
die zweite Steuerung (27) der Eingabevorrichtung für medizinische Geräte (5) eine Benachrichtigung sendet, ob eine Nahfeldkommunikation zwischen der ersten Nahfeldkommunikationskomponente (20) der pharmazeutischen Injektionsvorrichtung (1) und der zweiten Nahfeldkommunikationskomponente (38) der mobilen Kommunikationsvorrichtung (2) durchgeführt werden soll oder nicht.

2. Medizinisches Hilfssystem nach Anspruch 1, wobei die Kommunikation zwischen der ersten Kommunikationskomponente (20) der Eingabevorrichtung für medizinische Geräte (5) und der zweiten Kommunikationskomponente (26) der mobilen Kommunikationsvorrichtung (2) eine Nahfeldkommunikation ist.

3. Medizinisches Hilfssystem nach Anspruch 1, wobei die zweite Steuerung (27) die Anzeigekomponente (28) veranlasst, eine Nachricht anzuzeigen, die an die Eingabevorrichtung für medizinische Geräte (5) meldet, ob eine Nahfeldkommunikation zwischen der ersten Nahfeldkommunikationskomponente (20) der pharmazeutischen Injektionsvorrichtung (1) und der zweiten Nahfeldkommunikationskomponente (38) der mobilen Kommunikationsvorrichtung (2) durchzuführen ist oder nicht.

4. Medizinisches Hilfssystem nach Anspruch 1 oder 3, wobei die zweite Steuerung (27) die Anzeigekomponente (28) veranlasst, das Datum anzuzeigen, an dem die pharmazeutische Injektionsvorrichtung (1) mit den von der Eingabevorrichtung für medizinische Geräte (5) empfangenen Änderungsdaten für die Antriebsgröße des Antriebsmotors (15) geändert werden soll.

5. Medizinisches Hilfssystem nach Anspruch 4, wobei die zweite Steuerung (27) die Anzeigekomponente (28) veranlasst, eine Eingabetaste zum Ausführen der Aktualisierung der Änderungsdaten und des geplanten Datums der Änderung anzuzeigen.

6. Medizinisches Hilfssystem nach Anspruch 5, wobei die zweite Steuerung (27) die Anzeigekomponente (28) veranlasst, eine Löschtaste zum Abbrechen der Anzeige der Eingabetaste anzuzeigen.

7. Medizinisches Hilfssystem nach Anspruch 6, wobei, wenn die Nahfeldkommunikation nicht durch die erste Nahfeldkommunikationskomponente (20) und die zweite Nahfeldkommunikationskomponente (38) am geplanten Datum der Änderung ausgeführt wird, die zweite Steuerung (27) die Anzeigekomponente (28) veranlasst, eine Meldung anzuzeigen, die die Eingabevorrichtung für medizinische Geräte (5) darüber informiert, dass die Nahfeldkommunikation nicht ausgeführt wird.

8. Medizinisches Hilfssystem nach Anspruch 1,
wobei
die zweite Steuerung (27) der mobilen Kommunikationsvorrichtung (2) eine Benachrichtigung von der mobilen Kommunikationsvorrichtung (2) an die Eingabevorrichtung für medizinische Geräte (5) darüber sendet, ob die Änderungsdaten gespeichert werden sollen oder nicht.

9. Medizinisches Hilfsverfahren zum Ändern der Arzneimittelinjektionsmenge einer pharmazeutischen Injektionsvorrichtung (1), umfassend:
einen ersten Übertragungsschritt zum Übertragen von Änderungsdaten, die von einem Bediener eingegeben werden, der von einer Eingabevorrichtung für medizinische Geräte (5) erkannt wird, an eine mobile Kommunikationsvorrichtung (2);
einen zweiten Übertragungsschritt zum Übertragen der Änderungsdaten von der mobilen Kommunikationsvorrichtung (2) an die pharmazeutische Injektionsvorrichtung (1) mittels Nahfeldkommunikation, über eine erste Nahfeldkommunikationskomponente (20) der pharmazeutischen Injektionsvorrichtung (1) und eine zweite Nahfeldkommunikationskomponente (38) der mobilen Kommunikationsvorrichtung (2), so dass die Änderungsdaten übertragen werden können durch Bewegen der mobilen Kommunikationsvorrichtung (2) in die Nähe der pharmazeutischen Injektionsvorrichtung (1), so dass die pharmazeutische Injektionsvorrichtung (1) nicht in Echtzeit mit der mobilen Kommunikationsvorrichtung (2) verbunden sein muss;
einen Ausführungsschritt des Erkennens von Datenspeicherung in einem Speicher (19) und des Einstellens der Änderungsdaten an der pharmazeutischen Injektionsvorrichtung (1);
einen dritten Übertragungsschritt zum Übertragen einer Benachrichtigung an die Eingabevorrichtung für medizinische Geräte (5) darüber, ob eine Nahfeldkommunikation zwischen der ersten Nahfeldkommunikationskomponente (20) der pharmazeutischen Injektionsvorrichtung (1) und der zweiten Nahfeldkommunikationskomponente (38) der mobilen Kommunikationsvorrichtung (2) durchgeführt werden soll oder nicht;
einen vierten Übertragungsschritt zum Übertragen von Informationen, die anzeigen, dass die Änderungsdaten durch die Nahfeldkommunikation gespeichert wurden, von der pharmazeutischen Injektionsvorrichtung (1) zu der mobilen Kommunikationsvorrichtung (2); und
einen fünften Übertragungsschritt zum Übertragen vom Speichern oder Nicht-Speichern der Änderungsdaten von der mobilen Kommunikationsvorrichtung (2) zu der Eingabevorrichtung für medizinische Geräte (5).

## Revendications

1. Système d'assistance médicale comprenant:
un dispositif d'injection pharmaceutique (1) comprenant un boîtier de corps principal (6); un piston (9) qui pousse un médicament contenu dans ledit boîtier de corps principal (6) à travers une aiguille d'injection (10) et dans le corps d'un patient; un moteur d'entraînement (15) qui entraîne le piston (9); une première commande (14) qui est reliée au moteur d'entraînement (15); une mémoire (19) qui est reliée à ladite première commande (14); un composant de reconnaissance de stockage qui reconnaît que des données ont été stockées dans la mémoire (19); un premier composant de communication en champ proche (20) qui est relié à la première commande (14); et une batterie (22) qui alimente en courant le moteur d'entraînement (15), la première commande (14), la mémoire (19) et le premier composant de communication en champ proche (20);
un dispositif d'entrée d'équipement médical (5) ayant un composant de reconnaissance qui reconnaît un opérateur; un composant d'entrée qui entre des données de changement relatives à une quantité d'entraînement du moteur d'entraînement (15); et un premier composant de communication (39) qui envoie des données de changement relatives à la quantité d'entraînement du moteur d'entraînement (15) à un dispositif de communication mobile (2); et
ledit dispositif de communication mobile (2) ayant un deuxième composant de communication en champ proche (38) qui effectue une communication en champ proche avec le premier composant de communication en champ proche (20) du dispositif d'injection pharmaceutique (1); une deuxième commande (27) qui est reliée au deuxième composant de communication en champ proche (38); un composant d'affichage (23) qui est relié à la deuxième commande (27); et un deuxième composant de communication (26) qui est relié à la deuxième commande (27) et communique avec le dispositif d'entrée d'équipement médical (5),
dans lequel, avec le dispositif d'entrée d'équipement médical (5), les données de changement ne peuvent être entrées que par l'opérateur reconnu par le composant de reconnaissance,
le premier composant de communication (39) envoie au deuxième composant de communication (26) des données de changement relatives à la quantité d'entraînement du moteur d'entraînement (15) entrées par l'opérateur,
la première commande (14) du dispositif d'injection pharmaceutique (1) reçoit les données de changement relatives à la quantité d'entraînement du moteur d'entraînement (15) reçues par le deuxième composant de communication (26) du dispositif de communication mobile (2), à partir du deuxième composant de communication en champ proche (38) du dispositif de communication mobile (2) via le premier composant de communication en champ proche (20), de sorte que les données de changement puissent être transmises en déplaçant le dispositif de communication mobile (2) à proximité du dispositif d'injection pharmaceutique (1) de sorte que le dispositif d'injection pharmaceutique (1) ne doit pas être relié en temps réel au dispositif de communication mobile (2),
les données de changement relatives à la quantité d'entraînement du moteur d'entraînement (15) sont enregistrées dans la mémoire (19), ce stockage dans la mémoire (19) est reconnue,
les informations indiquant le stockage des données de changement sont renvoyées par le premier composant de communication en champ proche (20) au dispositif de communication mobile (2), les informations sont transmises du dispositif de communication mobile (2) au dispositif d'entrée d'équipement médical (5) avec le deuxième composant de communication (26) et le premier composant de communication (20), et
la deuxième commande (27) envoie au dispositif d'entrée d'équipement médical (5) une notification indiquant s'il doit ou non effectuer une communication en champ proche entre le premier composant de communication en champ proche (20) du dispositif d'injection pharmaceutique (1) et le deuxième composant de communication en champ proche (38) du dispositif de communication mobile (2).

2. Système d'assistance médicale selon la revendication 1, dans lequel la communication entre le premier composant de communication (20) du dispositif d'entrée d'équipement médical (5) et le deuxième composant de communication (26) du dispositif de communication mobile (2) est une communication en champ proche.

3. Système d'assistance médicale selon la revendication 1, dans lequel la deuxième commande (27) amène le composant d'affichage (28) à afficher un message qui signale au dispositif d'entrée d'équipement médical (5) s'il doit ou non effectuer une communication en champ proche entre le premier composant de communication en champ proche (20) du dispositif d'injection pharmaceutique (1) et le deuxième composant de communication en champ proche (38) du dispositif de communication mobile (2).

4. Système d'assistance médicale selon la revendication 1 ou 3, dans lequel la deuxième commande (27) amène le composant d'affichage (28) à afficher la date à laquelle le dispositif d'injection pharmaceutique (1) doit être modifié avec les données de changement pour la quantité d'entraînement du moteur d'entraînement (15) reçues dudit dispositif d'entrée d'équipement médical (5).

5. Système d'assistance médicale selon la revendication 4, dans lequel la deuxième commande (27) amène le composant d'affichage (28) à afficher une touche d'entrée pour effectuer la mise à jour des données de changement et de la date prévue du changement.

6. Système d'assistance médicale selon la revendication 5, dans lequel la deuxième commande (27) amène le composant d'affichage (28) à afficher une touche d'effacement pour effacer l'affichage de la touche d'entrée.

7. Système d'assistance médicale selon la revendication 6, dans lequel, lorsque la communication en champ proche n'est pas effectuée par le premier composant de communication en champ proche (20) et le deuxième composant de communication en champ proche (38) à la date prévue du changement, la deuxième commande (27) amène le composant d'affichage (28) à afficher un message informant le dispositif d'entrée d'équipement médical (5) que la communication en champ proche n'est pas effectuée.

8. Système d'assistance médicale selon la revendication 1,
dans lequel
la deuxième commande (27) du dispositif de communication mobile (2) envoie depuis le dispositif de communication mobile (2) vers le dispositif d'entrée d'équipement médical (5) une notification indiquant si les données de changement doivent être stockées ou non.

9. Procédé d'assistance médicale pour changer la quantité d'injection de médicament d'un dispositif d'injection pharmaceutique (1), comprenant:
une première étape de transmission consistant à transmettre des données de changement entrées par un opérateur reconnu au niveau d'un dispositif d'entrée d'équipement médical (5), à un dispositif de communication mobile (2);
une deuxième étape de transmission consistant à transmettre les données de changement depuis le dispositif de communication mobile (2) vers le dispositif d'injection pharmaceutique (1) au moyen d'une communication en champ proche via un premier composant de communication en champ proche (20) du dispositif d'injection pharmaceutique (1) et un deuxième composant de communication en champ proche (38) du dispositif de communication mobile (2), de sorte que les données de changement puissent être transmises en déplaçant le dispositif de communication mobile (2) à proximité du dispositif d'injection pharmaceutique (1), de sorte que le dispositif d'injection pharmaceutique (1) ne doit pas être relié en temps réel au dispositif de communication mobile (2);
une étape d'exécution consistant à reconnaître le stockage dans une mémoire (19) et à ajuster les données de changement au niveau du dispositif d'injection pharmaceutique (1);
une troisième étape de transmission consistant à transmettre au dispositif d'entrée d'équipement médical (5) une notification indiquant s'il doit ou non effectuer une communication en champ proche entre le premier composant de communication en champ proche (20) du dispositif d'injection pharmaceutique (1) et le deuxième composant de communication en champ proche (38) du dispositif de communication mobile (2);
une quatrième étape de transmission consistant à transmettre des informations depuis le dispositif d'injection pharmaceutique (1) vers le dispositif de communication mobile (2), qui indiquent que les données de changement ont été stockées par la communication en champ proche; et
une cinquième étape de transmission consistant à transmettre un stockage ou un non stockage des données de changement depuis le dispositif de communication mobile (2) vers le dispositif d'entrée d'équipement médical (5).
